(19) Europäisches Patentamt
European Patent Office
Office européen des brevets

(11) Publication number: **0 127 756**
**B1**

(12) **EUROPEAN PATENT SPECIFICATION**

(45) Date of publication of patent specification: **26.10.88**

(51) Int. Cl.[4]: **C 07 D 213/82**, C 07 D 213/85

(21) Application number: **84104452.2**

(22) Date of filing: **19.04.84**

(54) **Process for the preparation of 5-pyridyl-pyridine-2(1H)-ones.**

(30) Priority: **20.04.83 ES 521644**

(43) Date of publication of application:
**12.12.84 Bulletin 84/50**

(45) Publication of the grant of the patent:
**26.10.88 Bulletin 88/43**

(84) Designated Contracting States:
**CH DE FR GB IT LI NL**

(56) References cited:
**GB-A-2 070 606**
**US-A-4 225 715**

(73) Proprietor: **FABRICA DE PRODUCTOS QUIMICOS Y FARMACEUTICOS ABELLO, S.A.**
**C/ Julián Camarillo, 8**
**Madrid 17 (ES)**

(72) Inventor: **Gomez Parra, Vicente**
**Corazon de Maria, 33**
**28002 Madrid (ES)**
Inventor: **Gonzalez Hernandez, Pedro**
**C/ Máximo San Juan No. 10**
**Madrid (ES)**
Inventor: **Sanchez Alonso, Félix**
**C/ Canillas, 6**
**Madrid (ES)**
Inventor: **Torres Cebada, Tomás**
**C/Granada, 42**
**Madrid (ES)**

(74) Representative: **Abitz, Walter, Dr.-Ing. et al**
**Abitz, Morf, Gritschneder, Freiherr von Wittgenstein Postfach 86 01 09**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 127 756 B1**

Courier Press, Leamington Spa, England.

**Description**

This invention relates to improved processes for preparing 5-pyridyl-pyridine-2(1*H*)-one compounds.

Some 5-pyridyl-pyridine-2(1*H*)-one compounds are described as cardiotonically active drugs (see US—A—4,107,315; DE—A—3,944,568) and are prepared by condensing under classical conditions, preferred by employing sodium methoxide in methanol or dimethylformamide with only low to moderate yields and in a drastic basic media.

It is known from US—A—4,225,715 to react a 3-dimethylamino-2-pyridylacrolein compound with malonamide or with cyanoacetamide. Those reactions are carried out in a lower alkanol as solvent and an alkali lower alkoxide as basic condensing agent.

The first process according to the invention relates to the preparation of 5-pyridyl-3-carbamoyl-pyridine-2(1*H*)-one compounds of the general formula

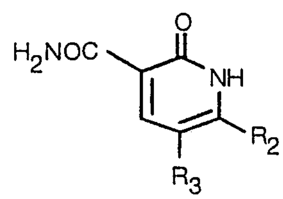

IV

wherein $R_2$ is a hydrogen atom or a $C_{1-8}$alkyl group, preferably $C_{1-6}$alkyl, most preferably $C_{1-4}$alkyl, and $R_3$ is a 4-, 3- or 2-pyridyl group. According thereto, a 3-dimethylaminoacrolein compound of the general formula

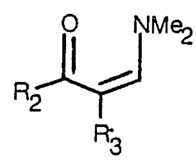

II

is reacted with malonamide both dissolved in chloroform, methylene chloride, acetonitrile or dimethylformamide and in the presence of a basic compound. The reaction is carried out in the presence of finely ground alkali or alkaline earth carbonate, bicarbonate or hydroxide and of an ammonium or phosphonium quaternary salt which performs as a phase-transfer catalyst (PTC). This process may be suitably conducted at a temperature within the range of about 20 to 160°C, continuously stirred at a rate higher than 200 r.p.m. and for a period within the range of about 24 to 72 hours to afford compounds of general formula IV which can be purified by recrystallization from a suitable solvent, although this is not necessary for them to be used in the next step.

The second process according to the invention relates to the preparation of 5-pyridyl-3-cyano-pyridine-2(1*H*)-one compounds of the general formula

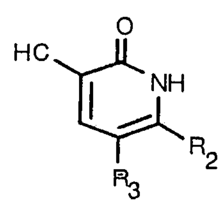

III

wherein $R_2$ is a hydrogen atom or a $C_{1-8}$alkyl group, preferably $C_{1-6}$alkyl, most preferably $C_{1-4}$alkyl, and $R_3$ is a 4-, 3- or 2-pyridyl group. In this process a compound of the general formula

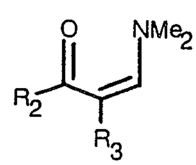

II

is reacted with cyanoacetamide in the presence of chloroform, methylene chloride, acetonitrile or dimethylformamide and in the presence of a basic compound. The reaction is carried out in the presence of finely ground alkali or alkaline earth carbonate, bicarbonate or hydroxide and of an ammonium or phosphonium quaternary salt which performs as a phase-transfer catalyst. This process may be suitably conducted at a temperature within the range of about 10 to 160°C, continuously stirred at a rate higher than 200 r.p.m. and for a period within the range of about 2 to 36 hours to afford compounds of general formula

III. These compounds can be purified by recrystallization from a suitable solvent, although this is not necessary to carry on with the synthesis.

The cyano group of those compounds can be partially hydrolyzed to a carbamoyl group following the usual procedures to afford the compounds of general formula IV.

The carboxamide compounds of general formula IV lead in the following step to the corresponding primary amine compounds following the usual procedures as depicted in the following scheme:

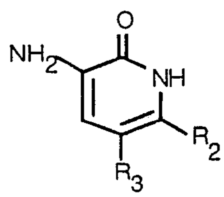

IV

for example by treatment with an alkaline hypohalide [Org. React., *3*, 267(1946)], by the action of hydrazine and further nitrosation and heat [Org. React., *3*, 337(1946)].

Some of the compounds prepared according to the invention have shown to be effective as antidysrhythmic agents, and some of them are useful as intermediates in the preparations of other derivatives with the therapeutic activity mentioned.

The following examples illustrate the processes used for preparing the 5-pyridyl-pyridine-2(1*H*)-ones, and are not intended to limit the scope of this invention.

Example 1

5-(4-pyridyl)-3-carbamoyl-pyridine-2(1*H*)-one

Into a 2-litre round-bottomed flask, provided with a mechanical stirrer, a reflux condenser and a calcium chloride tube, 2.5 L of acetonitrile, 100 g of 2-(4-pyridyl)-3-dimethylaminoacroleine, 64 g of malonoamide, 20 g of tetrabutylammonium bromide and 300 g of finely ground potassium carbonate are placed. The mixture is vigorously stirred at 400 to 600 r.p.m. and refluxed for 24 hours. Then 50 g of finely ground potassium hydroxide are added and stirring and refluxing continued for 4 hours more. The reaction mixture was evaporated to dryness "in vacuo", then 2 L of water are added and the mixture is distilled under reduced pressure affording a first fraction which contains the excess of acetonitrile, thus reducing the total mixture volume to approximately 2 L. This solution is kept at about 40 to 60°C and 50% sulphuric acid is added until pH = 7 whereupon a pale beige product precipitates. The precipitate is filtered under "vacuum", washed with water and dried in the air to yield 72 g (58%) of the title compound, m.p. > 300°C. Following a similar process and starting from suitable products of general formula II the following products, among others, are prepared:

5-(3-Pyridyl)-3-carbamoyl-pyridine-2(1*H*)-one: Yield 68%. m.p. > 300°C.

5-(4-Pyridyl)-3-carbamoyl-6-methyl-pyridine-2(1*H*)-one: Yield 62%. m.p. > 300°C.

Example 2

5-(4-pyridyl)-3-cyano-pyridine-2(1*H*)-one

Into a 3-litre round bottomed flask provided with a mechanical stirrer and a calcium chloride tube, 2.5 L of acetonitrile, 100 g of 2-(4-pyridyl)-3-dimethylaminoacroleine, 52.5 g of cyanoacetamide, 20 g of tetrabutylammonium bromide and 100 g of finely ground potassium hydroxide are added. The mixture is vigorously stirred at 400 to 600 r.p.m. at room temperature (water bath 20°C) for 4 hours. Then the reaction mixture is evaporated to dryness "in vacuo" and 2 L of water are added and distilled under reduced pressure to afford a first fraction distillation in which the excess of acetonitrile is contained to end up with a volume of approximately 2 L. The solution is kept at a temperature between 40 to 60°C and 50% sulphuric acid is added until pH = 7 whereupon a yellow solid product precipitates. This precipitate filtered under "vacuum", washed with water and dried in the air to afford 95 g (85%) of the title compound. m.p. > 300°C. Following a similar procedure and starting from suitable products of general formula II the following compounds, among others, are prepared:

5-(2-pyridyl)-3-cyano-pyridine-2(1*H*)-one: Yield 90%. m.p. > 300°C.

5-(4-pyridyl)-3-cyano-6-methyl-pyridine-2(1*H*)-one. Yield 82%. m.p. > 300°C.

5-(3-pyridyl)-3-cyano-pyridine-2(1*H*)-one: Yield 87%. m.p. > 300°C.

5-(3-pyridyl)-3-cyano-6-ethyl-pyridine-2(1*H*)-one: Yield 68%. m.p. > 300°C.

**Claims**

1. Process for preparing 5-pyridyl-3-carbamoyl-pyridine-2(1*H*)-one compounds of the general formula

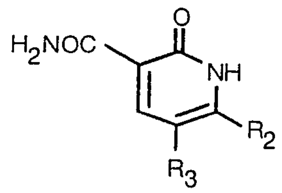

IV

wherein $R_2$ is a hydrogen atom or a $C_{1-8}$alkyl group and $R_3$ is a 4-, 3- or 2-pyridyl group, a 3-dimethylaminoacrolein compound of the general formula

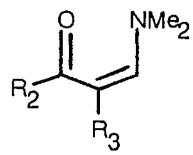

II

being reacted with malonamide both dissolved in chloroform, methylene chloride, acetonitrile or dimethylformamide and in the presence of a basic compound, characterized in that the reaction is carried out in the presence of finely ground alkali or alkaline earth carbonate, bicarbonate or hydroxide and of an ammonium or phosphonium quaternary salt.

2. Process for preparing 5-pyridyl-3-cyano-pyridine-2-(1*H*)-one compounds of the general formula

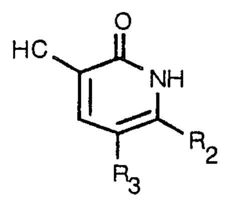

III

wherein $R_2$ is a hydrogen atom or a $C_{1-8}$alkyl group and $R_3$ is a 4-, 3- or 2-pyridyl group, a compound of the general formula

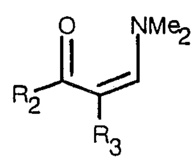

II

being reacted with cyanoacetamide in the presence of chloroform, methylene chloride, acetonitrile or dimethylformamide and in the presence of a basic compound, characterized in that the reaction is carried out in the presence of finely ground alkali or alkaline earth carbonate, bicarbonate or hydroxide and of an ammonium or phosphonium quaternary salt.

3. The process according to claim 1 wherein a reaction temperature of 20—160°C is used.

4. The process according to claim 2 wherein a reaction temperature of 10—160°C is used.

**Patentansprüche**

1. Verfahren zur Herstellung von 5-Pyridyl-3-carbamoyl-pyridin-2(1*H*)-on-Verbindungen der allgemeinen Formel

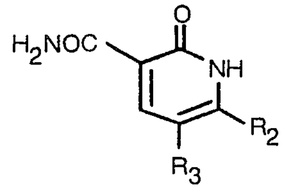

IV

worin $R_2$ ein Wasserstoffatom oder eine $C_{1-8}$-Alkylgruppe ist und $R_3$ eine 4-, 3- oder 2-Pyridylgruppe ist, durch Umsetzung einer 3-Dimethylaminoacrolein-Verbindung der allgemeinen Formel

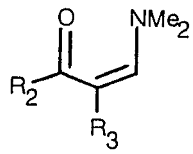

II

mit Malonamid, beide gelöst in Chloroform, Methylenchlorid, Acetonitril oder Dimethylformamid, und in Gegenwart einer basischen Verbindung, dadurch gekennzeichnet, dass die Umsetzung durchgeführt wird in Gegenwart von fein gemahlenem Alkali- oder Erdalkalicarbonat, -bicarbonat oder -hydroxid und eines quartären Ammonium- oder Phosphoniumsalzes.

2. Verfahren zur Herstellung von 5-Pyridyl-3-cyan-pyridin-2-(1*H*)-on-Verbindungen der allgemeinen Formel

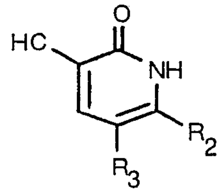

III

worin $R_2$ ein Wasserstoffatom oder eine $C_{1-8}$-Alkylgruppe ist und $R_3$ eine 4-, 3- oder 2-Pyridylgruppe ist, durch Umsetzung einer Verbindung der allgemeinen Formel

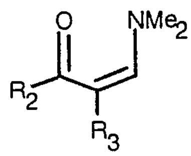

II

mit Cyanacetamid in Gegenwart von Chloroform, Methylenchlorid, Acetonitril oder Dimethylformamid und in Gegenwart einer basischen Verbindung, dadurch gekennzeichnet, dass die Umsetzung durchgeführt wird in Gegenwart von fein gemahlenem Alkali- oder Erdalkalicarbonat, -bicarbonat oder -hydroxid und eines quartären Ammonium- oder Phosphoniumsalzes.

3. Das Verfahren nach Anspruch 1, worin eine Reaktions-temperatur von 20—160°C verwendet wird.

4. Das Verfahren nach Anspruch 2, worin eine Reaktions-temperatur von 10—160°C verwendet wird.

**Revendications**

1. Procédé pour la préparation de composés 5-pyridyl-3-carbamoyl-pyridine-2(1*H*)-one de formule générale

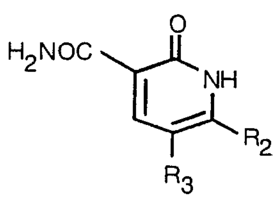

IV

dans laquelle $R_2$ est un atome d'hydrogène ou un groupe alkyle en $C_{1-8}$ et $R_3$ est un groupe 4-, 3- ou 2-pyridyle, dans lequel on fait réagir une 3-diméthylaminoacroléine de formule générale

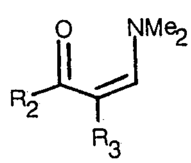

II

avec la malonamide, tous les deux étant dissous dans du chloroforme, du chlorure de méthylène, de l'acétonitrile ou du diméthylformamide, et en présence d'un composé basique, caractérisé en ce que l'on effectue la réaction en présence d'un carbonate, d'un bicarbonate ou d'un hydroxyde d'un métal alcalin ou d'un métal alcalino-terreux finement broyé et d'un sel quaternaire d'ammonium ou de phosphonium.

2. Procédé pour la préparation de composés 5-pyridyl-3-cyano-pyridine-2-(1*H*)-one de formule générale

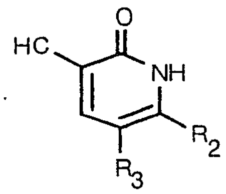

III

dans laquelle $R_2$ est un atome d'hydrogène ou un groupe alkyle en $C_{1-8}$ et $R_3$ est un groupe 4-, 3- ou 2-pyridyle, dans lequel on fait réagir un composé de formule générale

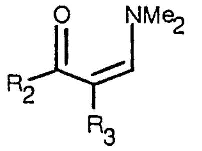

II

avec le cyano-acétamide, en présence de chloroforme, de chlorure de méthylène, d'acétonitrile ou de diméthylformamide et en présence d'un composé basique, caractérisé en ce que l'on effectue la réaction en présence d'un carbonate, d'un bicarbonate ou d'un hydroxyde d'un métal alcalin ou d'un métal alcalino-terreux finement broyé, et d'un sel quaternaire d'ammonium ou de phosphonium.

3. Procédé selon la revendication 1, dans lequel on utilise une température de réaction comprise entre 20 et 160°C.

4. Procédé selon la revendication 2, dans lequel on utilise une température de réaction comprise entre 10 et 160°C.